# EUROPEAN PATENT SPECIFICATION

(11) **EP 4 255 430 B1**
(45) Date of publication and mention of the grant of the patent: **29.04.2026**
(21) Application number: 21814820.3
(22) Date of filing: 02.12.2021
(51) Int. Cl.: A61K 31/47, A61P 31/14

(54) **8-HYDROXYQUINOLINE CYSTEINE PROTEASE INHIBITORS FOR USE IN THE PREVENTION AND/OR TREATMENT OF A CORONA VIRUS DISEASE**
8-HYDROXYCHINOLIN-CYSTEINPROTEASE-INHIBITOREN ZUR VERWENDUNG BEI DER VORBEUGUNG UND/ODER BEHANDLUNG EINER CORONAVIRUSERKRANKUNG
INHIBITEURS DE CYSTÉINE-PROTÉASE DE TYPE 8-HYDROXYQUINOLÉINE POUR LA PRÉVENTION ET/OU LE TRAITEMENT D'UNE MALADIE DUE AU CORONA VIRUS

(30) Priority: 04.12.2020 LU 102267
(43) Date of publication of application: 11.10.2023
(73) Proprietor: Univerza v Ljubljani, 1000 Ljubljana (SI); International Centre For Genetic Engineering And Biotechnology, 34149 Trieste (IT)
(72) Inventor: KOS, Janko, 1000 Ljubljana (SI); MITROVIC, Ana, 1000 Ljubljana (SI); SOSOC, Izidor, 1000 Ljubljana (SI); GOBEC, Stanislav, 1000 Ljubljana (SI); MARCELLO, Alessandro, 34149 Trieste (IT); BONOTTO, Rafaela, 34149 Trieste (IT)
(74) Representative: Zacco GmbH
(86) International application number: PCT/EP2021/083960
(87) International publication number: WO 2022/117724

(56) References cited:
- EP-A1- 2 353 599
- JOSHI SIDDHI ET AL: "Tackling SARS-CoV-2: proposed targets and repurposed drugs", vol. 12, no. 17, 22 June 2020 (2020-06-22), GB, pages 1579 - 1601, XP055826809, ISSN: 1756-8919, Retrieved from the Internet <URL:https://www.future-science.com/doi/pdfplus/10.4155/fmc-2020-0147> DOI: 10.4155/fmc-2020-0147
- SOSIC IZIDOR ET AL: "Cathepsin B inhibitors: Further exploration of the nitroxoline core", BIOORGANIC & MEDICINAL CHEMISTRY LETTERS,, vol. 28, no. 7, 15 April 2018 (2018-04-15), AMSTERDAM, NL, pages 1239 - 1247, XP055826636, ISSN: 0960-894X, DOI: 10.1016/j.bmcl.2018.02.042

## Description

### Technical field of the invention

The present invention generally relates to 8-hydroxyquinoline cysteine protease inhibitors, and more specifically to 5-nitro-8-hydroxyquinoline (nitroxoline) and derivatives thereof, for use in the prevention and/or treatment of a disease caused by a virus of the coronavirus subfamily, and in particular of a disease caused by the virus SARS-CoV-2.

### Background of the invention

Peptidases are enzymes catalysing the cleavage of peptide bond in polypeptides. According to their structure and catalytic mechanism they are divided into several clans and families. Lysosomal peptidases comprise the cathepsins, which are subdivided into three subgroups according to the active site amino acid residue: aspartyl (cathepsins D and E), serine (cathepsins A and G), and cysteine cathepsins. The latter constitute the largest cathepsin family, with 11 peptidases in humans belonging to clan CA, family C1a: cathepsins B, C/DPP1, F, H, K, L, O, S, W, V, and Z/X (Rawlings et al. 2018). Cathepsins were first considered as enzymes, responsible for the non-specific bulk proteolysis in the endosomal/lysosomal compartments (Turk, Turk, and Turk 2000). Later, a broad spectrum of their more specific roles was proposed, under both physiological and pathological conditions, including a role in virus infections (Simmons et al. 2013).

The role of cysteine proteases in the viral life cycle is particularly associated with the processing of viral polyprotein involved in virus replication and in the entry of the virus to the host cells. In the first process includes predominantly viral cysteine proteases whereas the latter employs various host proteases. In non-enveloped viruses (i.e., reoviruses), the virion is converted into an infectious subvirion particle via partial proteolysis, mediated by host cathepsins B, L, or S (Baer et al. 1999; Ebert et al. 2002; Golden et al. 2002; Jané-Valbuena et al. 2002; Golden et al. 2004). In the last decade, the proteolytic digestion by host endosomal cathepsins has been described during entry of enveloped viruses, such as the Ebola virus and corona viruses (CoVs) (Chandran et al. 2005; Simmons et al. 2013). In particular, CoVs have been extensively investigated during last decay regarding the role of viral and host cysteine peptidases in viral life cycle. Among host cysteine proteases cathepsin L is most commonly associated with the activation of viral glycoproteins. It is known to process the SARS-CoV spike protein as well as many proteins of other HCoVs, including MERS-CoV and HCoV-229E (Simmons et al. 2005; Shirato, Kawase, and Matsuyama 2013; Kawase et al. 2009). The cysteine peptidase cathepsin B can also be involved in virion entry and exhibits distinct properties compared to cathepsin L and other cathepsins; it generally has a higher pH optimum and, while preferring an aromatic P2 residue, can process dibasic substrates (Choe et al. 2006; Asbóth, Majer, and Polgár 1988). Cathepsin B was shown to play key roles in the lifecycle of the Ebola virus (Chandran et al. 2005), Nipah virus (Diederich, Thiel, and Maisner 2008), and feline CoV (Regan et al. 2008), catalytically activating viral membrane glycoproteins, which leads to fusion of the viral envelope with the endosomal membrane and virion release from endosomes into the cytoplasm (Nanbo et al. 2010; Gnirss et al. 2012).

For SARS-CoVs uptake to host cells, cathepsin L, rather than other cathepsins, is involved in receptor mediated endocytosis, in which the interaction with ACE2 is required (Simmons et al. 2005; Huang et al. 2006). Based on these observations, a three-step process of SARS-CoVs infection has been proposed: receptor binding, induced conformational changes in spike protein, and cathepsin L proteolysis within the endosomes (Simmons et al. 2005). However, for SARS-CoV-2 the spike protein was suggested to be processed by serine peptidase transmembrane peptidase/serine subfamily member 2 (TMPRSS2) resulting in membrane fusion and direct release of viral genetic material to the host cell. Nevertheless, recent studies show that serine peptidase inhibitors only partially blocked viral uptake and that both pathways should be involved in SARS-CoV-2 viral infection (Hoffmann et al. 2020).

Siddhi et al., Future Medicinal Chemistry 12 (2020) p1579-1601 reviews many different drug classes for possible applicability of all existing drugs for being useful in helping to combat the SARS-Cov2 pandemic which was evolving at the time of its publication.

### Summary of the invention

The objective of the present invention is thus to provide compounds which are effective in the prevention and/or treatment of a disease caused by a virus of the coronavirus subfamily, and in particular of a disease caused by the virus SARS-CoV-2.

This objective has been met by the present inventors who have surprisingly found that 8-hydroxyquinoline cysteine protease inhibitors of general formula (I) exhibit superior efficacy in the prevention and/or treatment of an infection caused by a corona virus, such as SARS-CoV-2. Particularly, the present inventors have shown that the highest activity against SARS-CoV-2 in the engineered Huh 7 cells expressing a human ACE2 (Huh7-hACE2 cells) was obtained by Nitroxoline: (5-nitro-8-hydroxyquinoline) and Compound 17: (2-{[(8-hydroxy-5-nitroquinoline-7-yl)methyl]amino}-acetonitrile), which have been previously identified as potent reversible cathepsin B inhibitors. The effect was significantly stronger compared to general cysteine peptidase inhibitor E64 as well as the effect of cathepsin L inhibitor CLIK 148. Another 8-hydroxyquinoline derivative Compound 3: (7-[(4-methylpiperidin-1yl)methyl]-5-nitroquinolin-8-ol), previously identified as an inhibitor of cathepsins B, H, and L, exhibited stronger effect than CLIK 148, but weaker than Nitroxoline and Compound 17. From these results it is evident that cathepsin B inhibitors, in particular 8-hydroxyquinoline derivatives inactivate host and/or viral proteases, involved in SARS-CoV-2 cell entry and viral replication. Contrary to some previous reports, it is surprising that cathepsin B and cathepsin B-like proteases are crucial for SARS-CoV-2 infection and replication. Nitroxoline, used in clinical practice for treatment of urinary tract infections, expresses its antibiotics activity as a metal ion chelator. Synergistic activity of cathepsin B inhibition and metal ion chelation could result in strong potential of Nitroxoline and other 8-hydroxyquinoline cysteine protease inhibitors against SARS-CoV-2.

The present invention thus provides in a first aspect a compound having general formula (**I**) 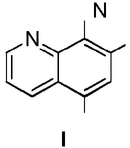 wherein:
R¹ is -H, -NO₂ or C₁₋₆ branched or linear alkyl optionally substituted with one or more substituents independently selected from the group of R⁵, -NO₂, -NH₂, 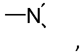 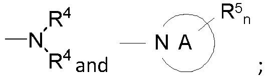
R² is -H, 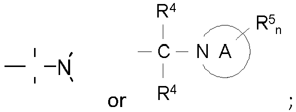
wherein A is a saturated or an unsaturated 3 to 8-membered heterocycle optionally comprising one or more further heteroatoms selected from N, O, S, and P, the heterocycle being optionally substituted with one or more substituents independently selected from R⁵, wherein n = 0 to 5;
R³ is -H, C₁₋₆ branched or linear alkyl optionally substituted with one or more substituents independently selected from R⁵, C₃-C₆ cycloalkyl optionally substituted with one or more substituents independently selected from R⁵;
R⁴ is -H; C₁₋₆ branched or linear alkyl optionally substituted with one or more substituents independently selected from R⁵ and optionally comprising one or more heteroatoms selected from N, S, O, P and optionally substituted with -N(R³)₂, C₃-C₆ cycloalkyl optionally comprising one or more heteroatoms selected from N, S, O, P and optionally substituted with -N(R³)₂, aryl optionally substituted with one or more substituents independently selected from R⁵, heteroaryl optionally substituted with one or more substituents independently selected from R⁵;
R⁵ is -H, halo, -NH₂, -OH, -CN, -NO₂, -COOH, -CONR⁴R⁴, -SO₂NR⁴R⁴, - SO₂R⁴, -CF₃, -NHSO₂R⁴, -NHCOR⁴, C₁₋₆ branched or linear alkyl, C₁₋₆ alkoxy or -SR⁴;
wherein multiple occurrences of any of R³, R⁴ and R⁵ in the structure means that the respective residues are independently selected from the respective groups defined above; or a pharmaceutically acceptable salt, hydrate or solvate thereof;
for use in the prevention and/or treatment of a disease caused by a virus of the coronavirus subfamily

The present invention provides in further aspect a pharmaceutical composition, which comprises a compound having general formula (I) as detailed herein and a pharmaceutically acceptable carrier, excipient and/or diluent, for use in the prevention and/or treatment of a disease caused by a virus of the coronavirus subfamily.

### Brief description of the figures

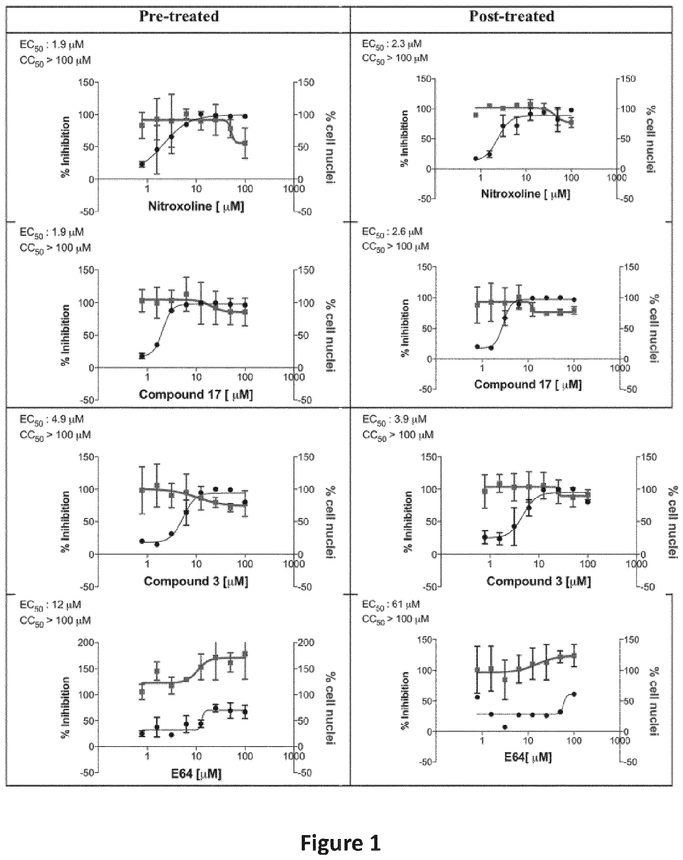

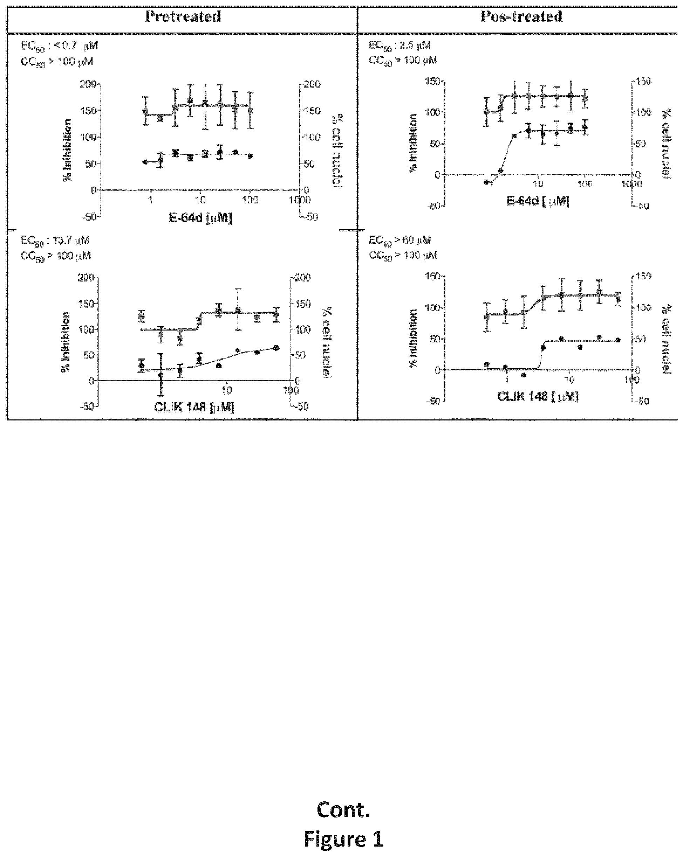

**Figure 1****:** Antiviral efficacy of compounds against SARS-CoV-2 was tested at pre-treated (left panels) and post-treated (right panels) conditions. The antiviral activity of compounds was evaluated by High Content Assay infecting Huh7-hACE2 cells in presence of increasing concentration of compounds. Percent of nuclei were quantified in parallel in the same assay to assess the compound cytotoxicity (Milani et al.,2020). The percentage infectivity inhibition (black dots) were normalized with the average number of infection ratio of infected wells, containing 1% DMSO and noninfected wells containing 1% DMSO. Percentage of nuclei (blue squares) were calculated by comparing the average number of nuclei of non-infected wells at the presence of 1% DMSO. Error bars represent the standard deviation (SD) of 2 independent experiments. For each panel EC₅₀ and CC₅₀ were calculated based on non -linear regression analysis and curve fitting parameters to provide relative values for inhibition of viral infection and cytotoxicity, respectively.

The present invention is now described in more detail below.

### Detailed description of the invention

As noted above, the present inventors have surprisingly found that 8-hydroxyquinoline cysteine protease inhibitors of general formula (I) exhibit superior efficacy in the prevention and/or treatment of an infection caused by a corona virus, such as SARS-CoV-2. Particularly, the present inventors have shown that the highest activity against SARS-CoV-2 in the Huh7-hACE2 cells line was obtained by Nitroxoline: (5-nitro-8-hydroxyquinoline) and Compound 17: (2-{[(8-hydroxy-5-nitroquinoline-7-yl)methyl]amino}-acetonitrile), which have been previously identified as potent reversible cathepsin B inhibitors. The effect was significantly stronger compared to general cysteine peptidase inhibitor E64 as well as the effect of cathepsin L inhibitor CLIK 148. Another 8-hydroxyquinoline derivative Compound 3: (7-[(4-methylpiperidin-1yl)methyl]-5-nitroquinolin-8-ol), previously identified as an inhibitor of cathepsins B, H, and L, exhibited stronger effect than CLIK 148, but weaker than Nitroxoline and Compound 17. From these results it is evident that cathepsin B inhibitors, in particular 8-hydroxyquinoline derivatives are able to inactivate host and/or viral proteases, involved in SARS-CoV-2 cell entry and viral replication. Contrary to some previous reports, it is surprising that cathepsin B and cathepsin B-like proteases are crucial for SARS-CoV-2 infection and replication and that peptidase-directed antiviral therapy should include various types of inhibitors, in particular cathepsin B inhibitors.

Nitroxoline, used in clinical practice for treatment of urinary tract infections, expresses its antibiotics activity as a metal ion chelator. Synergistic activity of cathepsin B inhibition and metal ion chelation could result in strong potential of Nitroxoline and other 8-hydroxyquinoline cysteine protease inhibitors against SARS-CoV-2. The other advantage of Nitroxoline and other 8-hydroxyquinoline derivatives in accordance of the invention is that they are reversible inhibitors in contrast to the other peptidase inhibitors, tested for antiviral effect for corona viruses. Reversible inhibition can provide better pharmacokinetic/pharmacodynamic properties over irreversible one and result in lower off-target effects.

Thus, the present invention provides in a first aspect a compound having general formula (**I**) 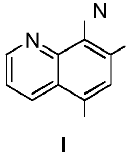 wherein:
R¹ is -H, -NO₂ or C₁₋₆ branched or linear alkyl optionally substituted with one or more substituents independently selected from the group of R⁵, -NO₂, -NH₂, 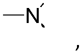 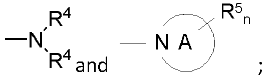
R² is -H, 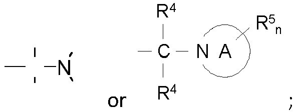
wherein A is a saturated or an unsaturated 3 to 8-membered heterocycle optionally comprising one or more further heteroatoms selected from N, O, S, and P, the heterocycle being optionally substituted with one or more substituents independently selected from R⁵, wherein n = 0 to 5;
R³ is -H, C₁₋₆ branched or linear alkyl optionally substituted with one or more substituents independently selected from R⁵, C₃-C₆ cycloalkyl optionally substituted with one or more substituents independently selected from R⁵;
R⁴ is -H; C₁₋₆ branched or linear alkyl optionally substituted with one or more substituents independently selected from R⁵ and optionally comprising one or more heteroatoms selected from N, S, O, P and optionally substituted with -N(R³)₂, C₃-C₆ cycloalkyl optionally comprising one or more heteroatoms selected from N, S, O, P and optionally substituted with -N(R³)₂, aryl optionally substituted with one or more substituents independently selected from R⁵, heteroaryl optionally substituted with one or more substituents independently selected from R⁵;
R⁵ is -H, halo, -NH₂, -OH, -CN, -NO₂, -COOH, -CONR⁴R⁴, -SO₂NR⁴R⁴, - SO₂R⁴, -CF₃, -NHSO₂R⁴, -NHCOR⁴, C₁₋₆ branched or linear alkyl, C₁₋₆ alkoxy or -SR⁴;
wherein multiple occurrences of any of R³, R⁴ and R⁵ in the structure means that the respective residues are independently selected from the respective groups defined above;
or a pharmaceutically acceptable salt, hydrate or solvate thereof;
for use in the prevention and/or treatment of a disease caused by a virus of the coronavirus subfamily.

According to some embodiments, R¹ is -NO₂.

According to some embodiments, R¹ is C₁₋₆ branched or linear alkyl.

According to some embodiments, R² is -H or

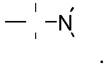

The compounds of general Formula (I) includes compounds having R² being -H, morpholinomethyl, pyrrolidinomethyl, (4-methylpiperazin-1-yl)methyl, (4-hydroxypiperidin-1-yl)methyl, (4-methypiperidin-1-yl)methyl, (4-carboxypiperidin-1-yl)methyl, or (3-hydroxypiperidin-1-yl)methyl.

According to some embodiments, the compound of formula (I) is selected from the group consisting of 5-nitro-8-hydroxyquinoline, 7-(Morpholinomethyl)-5-nitro-8-hydroxyquinoline, 7-(Pyrrolidinomethyl)-5-nitro-8-hydroxyquinoline, 7-((4-Methylpiperazin-1-yl)methyl)-5-nitro-8-hydroxyquinoline, 7-((4-Hydroxypiperidin-1-yl)methyl)-5-nitro-8-hydroxyquinoline, 7-((4-Methypiperidin-1-yl)methyl)-5-nitro-8-hydroxyquinoline, 7-((4-Carboxypiperidin-1-yl)methyl)-5-nitro-8-hydroxyquinoline and 7-((3-Hydroxypiperidin-1-yl)methyl)-5-nitro-8-hydroxyquinoline.

According to some embodiments, the compound of formula (I) is 5-nitro-8-hydroxyquinoline.

According to some embodiments, the compound of formula (I) is 7-(Morpholinomethyl)-5-nitro-8-hydroxyquinoline.

According to some embodiments, the compound of formula (I) is 7-(Pyrrolidinomethyl)-5-nitro-8-hydroxyquinoline.

According to some embodiments, the compound of formula (I) is 7-((4-Methylpiperazin-1-yl)methyl)-5-nitro-8-hydroxyquinoline.

According to some embodiments, the compound of formula (I) is 7-((4-Hydroxypiperidin-1-yl)methyl)-5-nitro-8-hydroxyquinoline.

According to some embodiments, the compound of formula (I) is 7-((4-Methypiperidin-1-yl)methyl)-5-nitro-8-hydroxyquinoline.

According to some embodiments, the compound of formula (I) is 7-((4-Carboxypiperidin-1-yl)methyl)-5-nitro-8-hydroxyquinoline.

According to some embodiments, the compound of formula (I) is 7-((3-Hydroxypiperidin-1-yl)methyl)-5-nitro-8-hydroxyquinoline.

"Alkyl", as used herein, shall be understood to relate to a branched or linear alkyl group with 1 to 20, preferably 1 to 10, most preferred 1 to 5 or 1 to 3 carbon atoms. Linear alkyl groups are preferred.

A "heterocycle", as used herein, shall be understood to relate to cyclic moieties comprising 2 to 7 carbon atoms in addition to at least one heteroatom in the cycle. Preferred heterocycles comprise 2 to 5, or 2 to 4 carbon atoms. Preferred heteroatoms are N, S, O, P. Preferred heterocycles of the invention comprise a single heteroatom.

"Branched alkyl", as used herein, shall be understood to relate to a branched alkyl group with 1 to 20, preferably 1 to 10, most preferred 1 to 5 or 1 to 3 carbon atoms.

"Aryl", as used herein, shall be understood to relate to any functional group or substituent derived from a simple aromatic ring, may it be phenyl, thiophene, indolyl, etc (according to IUPAC nomenclature). Preferred aryl groups are phenyl, C₆H₅; tolyl, CH₃C₆H₄; or xylyl, (CH₃)₂C₆H₃. Preferred aryl groups of the invention comprise 3 to 6 membered carbon cycles, most preferred 6 membered carbon cycles. Aryl groups of the invention may comprise a single aromatic cycle, but may also comprise two, or three, or more conjugated aromatic cycles. A single aromatic cycle is preferred.

"Heteroaryl" shall be understood to relate to aryl comprising a heteroatom in at least one of the aromatic cycles.

Methods for the preparation of compounds of formula (I) have been described in the patent and scientific literature, such as in WO 2010/034490, (Voronin et al. 1976; Mirković et al. 2011; Sosič et al. 2013; Mitrović et al. 2017; Sosič et al. 2018) the content of which is hereby incorporated by reference.

The present invention includes all hydrates, solvates, complexes, polymorphs and prodrugs of the compounds of formula (I). Prodrugs are any covalently bonded compounds which release the active parent drug according to formula (I) *in vivo.*

All forms of isomers resulting from the presence of a chiral center in the inventive compounds, including enantiomers and diastereomers, are intended to be covered herein. The inventive compounds may be used as a racemic mixture, an enantiomerically enriched mixture, or the racemic mixture may be separated using well-known techniques and an individual enantiomer may be used alone.

In the event that a compound of formula (I) may exist in tautomeric forms, each tautomeric form is considered as being included within this invention whether existing in equilibrium or predominantly in one form.

The present invention provides a pharmaceutical composition which comprises a compound of formula (I) and a pharmaceutically acceptable carrier, excipient and/or diluent. Furthermore, the compound of formula (I) can also be used in the preparation of a medicament.

The present invention provides a pharmaceutical composition which comprises a compound having general formula (I) and a pharmaceutically acceptable carrier, excipient and/or diluent, for use in the prevention and/or treatment of a disease caused by a virus of the coronavirus subfamily.

In some embodiments, the disease is caused by a corona virus selected from severe acute respiratory syndrome related coronavirus, Middle East respiratory syndrome related coronavirus, HCoV-NL63, HCoV-HKU1, HCoV-229E and HCoV-OC43.

In some embodiments, the disease is caused by SARS-CoV-2.

In some embodiments, the disease is COVID-19.

In some embodiments, the pharmaceutical composition is for use in the prevention and/or treatment of said disease in a human.

Suitable pharmaceutically acceptable carriers, excipients and diluents are well-known to the skilled person, and have been described in the literature, such as in Remington's Pharmaceutical Sciences, the Handbook of Pharmaceutical Additives or the Handbook of Pharmaceutical Excipients. Non-limiting examples of suitable pharmaceutically acceptable excipients include diluents, fillers, binding agents, disintegrating agents, lubricants, fluidizers, granulating agents, coating materials, wetting agents, solvents, co-solvents, suspending agents, emulsifying agents, sweeting agents, flavoring agents, odor masking agents, coloring agents, anti-caking agents, chelating agents, plasticisers, viscosifiers, antioxidants, antiseptics, stabilizing agents, surfactants and buffer agents.

Pharmaceutical preparations containing a compound of formula (I) may be formulated as solutions or lyophilized powders for parenteral administration. In the latter case, powders may be reconstituted by addition of a suitable diluent or other pharmaceutically acceptable carrier prior to use. The liquid preparation may be buffered, isotonic, aqueous solution. Examples of suitable diluents are normal isotonic saline, standard 5 % dextrose in water, or buffered sodium or ammonium acetate solution. Such formulation is especially suitable for parenteral administration, but may be also used for oral administration or contained in a metered dose inhaler or nebulizer for insufflation. It may be desirable to add excipients such as polyvinylpyrrolidone, gelatin, hydroxy cellulose, acacia, polyethylene glycol, mannitol, sodium chloride, or sodium citrate.

Alternatively, a compound of formula (I) may be encapsulated, tableted, or prepared in an emulsion or syrup for oral administration. Pharmaceutically acceptable solid or liquid carriers may be added to enhance or stabilize the composition, or to facilitate preparation of the composition. Solid carriers include starch, lactose, calcium sulfate dihydrate, terra alba, magnesium stearate or stearic acid, talc, pectin, acacia, agar or gelatin. Liquid carriers include syrup, peanut oil, olive oil, saline and water. The carrier may also include a sustained release material such as glyceryl monostearate or glyceryl distearate, alone or with a wax. The amount of solid carrier may vary, but will preferably be between about 20 mg to about 1 g per dosage unit. The pharmaceutical preparations are made following the conventional pharmaceutical techniques, such as milling, mixing, granulating, and compressing when necessary for tablet forms; or milling, mixing and filling for hard gelatin capsule forms. When a liquid carrier is used, the preparation will be in the form of syrup, elixir, emulsion or an aqueous or a non-aqueous suspension. Such a liquid formulation may be administered directly or filled in a soft gelatin capsule or formulated as an aerosol.

For rectal administration, the compound of this invention may also be combined with excipients, such as cocoa butter, glycerin, gelatin or polyethylene glycols and moulded into a suppository.

The compounds of the present invention may be used for preventing and/or treating a disease caused by a virus of the coronavirus subfamily in a subject in need, comprising administering a therapeutically effective amount of a compound of formula (I) as defined herein or of a pharmaceutical composition as defined herein to a subject in need.

In accordance with this invention, an effective amount of a compound of formula (I) is administered to inhibit the cysteine peptidase cathepsin B. The dosage amount can be further modified according to the type of administration of the compound. For example, for acute therapy, parenteral administration of the compound of formula (I) is preferred. An intravenous infusion of the compound in 5 % dextrose in water or normal saline, or similar formulation with suitable excipients is most effective, although an intramuscular bolus injection is also useful. Typically, the parenteral dose will be about 0.01 to about 100 mg/kg, in a manner to maintain the concentration of drug in the plasma at a concentration effective to inhibit cathepsin B. The compound is administered one to four times daily at a level to achieve a total daily dose of about 0.4 to about 400 mg/kg/day. The precise amount of the inventive compound which is therapeutically effective, and the route by which such compound is best administered, is readily determined by one of the ordinary skill in the art by comparing blood levels of the agent to the concentration required to have a therapeutic effect.

Prodrugs of the compound of formula (I) may be prepared by any suitable method. Where the prodrug moiety is a ketone functionality, specifically ketals and/or hemiacetals, the conversion may be effected in accordance with conventional methods.

The compounds of formula (I) may be administered orally to the patient, in a manner such that the concentration of the drug is sufficient to achieve any therapeutic indication as disclosed herein. Typically, a pharmaceutical composition containing the compound is administered at an oral dose of between about 0.1 to about 50 mg/kg in a manner consistent with the condition of the patient.

As noted above, the compound of formula (I), the pharmaceutical composition, the medicament and the method of the presented invention are for use in the prevention and/or treatment of a disease caused by a virus of the coronavirus subfamily.

According to some embodiments, the disease is caused by human corona virus.

According to some embodiments, the disease is caused by a corona virus selected from severe acute respiratory syndrome related coronavirus, Middle East respiratory syndrome related coronavirus, HCoV-NL63, HCoV-HKU1, HCoV-229E and HCoV-OC43.

According to some embodiments, the disease is caused by SARS-CoV-2.

According to some embodiments, the disease is SARS.

According to some embodiments, the disease is COVID-19.

According to some embodiments, the subject in need of prevention and/or treatment is a mammal.

According to some embodiments, the subject in need of prevention and/or treatment is a human.

Having generally described this invention, a further understanding can be obtained by reference to certain specific examples, which are provided herein for purposes of illustration only, and are not intended to be limiting unless otherwise specified.

### Examples

### Selected inhibitors and controls:

1. **Nitroxoline:** (5-nitro-8-hydroxyquinoline). Specific reversible inhibitor of cathepsin B
2. **Compound 17:** 2-{[(8-hydroxy-5-nitroquinoline-7-yl)methyl]amino}-acetonitrile. Specific reversible inhibitor of cathepsin B
3. **Compound 3:** 7-[(4-methylpiperidin-1yl)methyl]-5-nitroquinolin-8-ol. Reversible inhibitor of cathepsins B, H, and L
4. **E64** ([L-trans-epoxysuccinyl-L-leucylamido(4-guanidino)butanel). General irreversible inhibitor of cysteine peptidases.
5. **E64d** (2S,3S)-*trans*-Epoxysuccinyl-L-leucylamido-3-methylbutane ethyl ester, (Aloxistatin). Cell-permeable and irreversible broad-spectrum cysteine protease inhibitor.
6. **CLIK 148** *N*-{L-3-*trans*-[2-(pyridin-2-yl)ethylcarbamoyl]-oxirane-2-carbonyl}-L-Phe dimethylamide; irreversible inhibitor of cathepsin L
7. **Positive control:** Infected cells treated with 50 µM 7-hydroxychloroquine
8. **Negative control:** Infected cells, containing final concentration of DMSO (1%)

### Methods

**Cells and virus:** Huh 7 cells (Hepatoma cell line) expressing the human ACE2 receptor was cultivated in DMEM (Gibco) supplemented with 10% fetal bovine serum (Gibco). SARS-CoV-2 isolate ICGEB_FVG_S5 (Licastro et al. 2020) was grown and quantified on Vero E6 cells.

**Dose response assay:** Compounds were prepared in 2-fold serial dilutions (8 points dilutions) in DMSO, and then diluted 16x in PBS 1X in an intermediate plate. Finally, compounds were transferred to the 96 well assay plate containing cells and virus suspension (6x in growth medium, final dilution 100x).

**High Content Assay:** Huh 7- ACE2 cells were seeded in a 96 wells plate, at 8x10³ cells/well density and incubated at 37 °C overnight. Compounds were tested in two conditions; (a) pre-treated, 3 hours prior infection compounds were transferred to the plate; (b) post treated, compounds were transferred to the plate immediately after infection. In all conditions, cells were infected with SARS-CoV-2 at 0.1 MOI. Controls included: infected cells treated with 50 µM of Hydroxychloroquine as well as non-infected cells treated with vehicle (1 % DMSO). Negative controls were infected cells treated with vehicle. Plates were incubated for 20 h at 37°C, and then fixed with 4% PFA for 15 min at room temperature and washed twice with PBS 1x. Cells were treated with 0,1% of Triton-X for 15 min, followed incubation for 30 min in blocking buffer (PBS containing 1% of bovine serum albumin - BSA). Then, a primary recombinant monoclonal Spike antibody (CR3022) was diluted in blocking buffer and incubated for 2 h at 37°C (Rajasekharan et al. 2020). Cells were washed 2 times in PBS 1X and incubated with the secondary antibody (anti-mouse Alexa 488) plus DAPI for 1 h at 37°C. Each plate was washed twice with PBS 1x. All plates were filled up with 150 µl of PBS/well. Digital images were acquired using a high content imaging system, the Operetta (Perkin Elmer). The digital images were taken from 9 different fields of each well at 20× magnification. Total number of cells and the number of infected cells were analysis using Columbus Image Data Storage and Analysis System (Perkin Elmer).

**Data normalization and analysis:** Infection ratio was defined as a ratio between (i) the total number of infected cells, and (ii) the total number of cells. Data were normalized with the negative (DMSO-treated, infected cells) and positive (infected cells treated with 50 µM Hydroxychloroquine) controls. Percentage inhibition was calculated based on infection ratio values with the formula: (1-( infection ratio samples - Avarage (Av) infection ratio of positive control) /(Av. infection ratio of negative control - Av. infection ratio of positive control)) x100. Percentage of nuclei was calculated from values of cell number with the formula: Cell number test sample/Avg. cell number of positive control) × 100. Values were plotted against dilutions expressed as antilog. The half maximal effective concentration (EC₅₀) and the half maximum cytotoxic concentration (CC₅₀) were calculated using GraphPad Prism Version 7.

### Results:

Results of the impact of cysteine peptidase inhibitors on SARS-CoV-2 viral activity and on cell viability are presented in Figure 1.

Antiviral activity is presented as % of inhibition, based on infection ratio values. The half maximal effective concentration (EC₅₀) is a relative measure of the effectiveness of the inhibitor. 50 µM 7-hydroxychloroquine as a positive control represents 100% inhibition.

Percentage of nuclei represents the impact of cysteine peptidase inhibitors on cell viability. Half maximum cytotoxic concentration (CC₅₀) is a relative measure of the cytotoxicity of the inhibitor.

It is evident that none of the inhibitors exhibited a significant cytotoxicity within the tested concentration range - CC₅₀< 100 µM.

Tests were performed at pre-treated conditions (compounds were transferred to the assay plate 3 hours prior infection) and post-treated conditions (compounds were transferred to the plate immediately after infection). In all cases except for compound 3 the effect (EC₅₀) was stronger at pre-treated conditions, suggesting the importance of cysteine peptidases for viral entry.

Nitroxoline and compound 17, both inhibitors of cathepsin B, reached 100% inhibition and exhibit a strong anti viral effect with EC₅₀ 1.9 µM at pre-treated conditions. The EC₉₀ , the effective concentration capable to inhibit 90% of infection, showed values < 10 µM, with 7.9 µM and 3.3 µM for Nitroxoline and Compound 17 respectively. They are followed by compound 3 with EC₅₀4.9 µM, which also reached 100% inhibition with EC₉₀ of 10 µM.

E64d, a general irreversible cell permeable inhibitor of cysteine proteases exhibited EC₅₀0.7 µM, but it did not reach 100% inhibition. E64d has been shown previously to reduce viral entry in MERS CoV (Pišlar et al. 2020).

Other two inhibitors tested, E64 and CLIK 148, exhibited EC₅₀at higher concentrations and they did not reach 100% inhibition.

### Conclusions

The results show that specific cathepsin B inhibitors, based on 8-hydroxyquinoline structure, such as nitroxoline and compound 17, effectively inhibit SARS-CoV-2 virus entry to host cells and its replication. The effect of compound 3, also possessing 8-hydroxyquinoline structure, but inhibiting besides cathepsin B also cathepsins H and L is also strong, but lower from that of nitroxoline and compound 17. Specific cathepsin L inhibitor CLIK 148 was less effective and did not reach 100% inhibition. The same holds for general inhibitors of cysteine proteases E64 and E64d.

**Taken together, 8-hydroxyquinoline based specific reversible inhibitors of cathepsin B are potent inhibitors of SARS-CoV-2 virus entry and replication and have a potential for treatment of Covid-19 and other corona virus related diseases.**

### List of references cited in the description

Asbóth, B, Z Majer, and L Polgár. 1988. "Cysteine Proteases: The S2P2 Hydrogen Bond Is More Important for Catalysis than Is the Analogous S1P1 Bond." FEBS Letters 233 (2): 339-41. https://doi.org/10.1016/0014-5793(88)80455-1.
Baer, G S, D H Ebert, C J Chung, A H Erickson, and T S Dermody. 1999. "Mutant Cells Selected during Persistent Reovirus Infection Do Not Express Mature Cathepsin L and Do Not Support Reovirus Disassembly." Journal of Virology 73 (11): 9532-43. https://doi.org/10.1128/JVI.73.11.9532-9543.1999.
Chandran, Kartik, Nancy J Sullivan, Ute Felbor, Sean P Whelan, and James M Cunningham. 2005. "Endosomal Proteolysis of the Ebola Virus Glycoprotein Is Necessary for Infection." Science (New York, N.Y.) 308 (5728): 1643-45. https://doi.org/10.1126/science.1110656.
Choe, Youngchool, Francesco Leonetti, Doron C Greenbaum, Fabien Lecaille, Matthew Bogyo, Dieter Brömme, Jonathan A Ellman, and Charles S Craik. 2006. "Substrate Profiling of Cysteine Proteases Using a Combinatorial Peptide Library Identifies Functionally Unique Specificities." The Journal of Biological Chemistry 281 (18): 12824-32. https://doi.org/10.1074/jbc.M513331200.
Diederich, Sandra, Lena Thiel, and Andrea Maisner. 2008. "Role of Endocytosis and Cathepsin-Mediated Activation in Nipah Virus Entry." Virology 375 (2): 391-400. https://doi.org/10.1016/j.virol.2008.02.019.
Ebert, Daniel H, Jan Deussing, Christoph Peters, and Terence S Dermody. 2002. "Cathepsin L and Cathepsin B Mediate Reovirus Disassembly in Murine Fibroblast Cells." The Journal of Biological Chemistry 277 (27): 24609-17. https://doi.org/10.1074/jbc.M201107200.
Gnirss, Kerstin, Annika Kühl, Christina Karsten, Ilona Glowacka, Stephanie Bertram, Franziska Kaup, Heike Hofmann, and Stefan Pöhlmann. 2012. "Cathepsins B and L Activate Ebola but Not Marburg Virus Glycoproteins for Efficient Entry into Cell Lines and Macrophages Independent of TMPRSS2 Expression." Virology 424 (1): 3-10. https://doi.org/10.1016/j.virol.2011.11.031.
Golden, Joseph W, Jessica A Bahe, William T Lucas, Max L Nibert, and Leslie A Schiff. 2004. "Cathepsin S Supports Acid-Independent Infection by Some Reoviruses." The Journal of Biological Chemistry 279 (10): 8547-57. https://doi.org/10.1074/jbc.M309758200.
Golden, Joseph W, Jessica Linke, Stephen Schmechel, Kara Thoemke, and Leslie A Schiff. 2002. "Addition of Exogenous Protease Facilitates Reovirus Infection in Many Restrictive Cells." Journal of Virology 76 (15): 7430-43. https://doi.org/10.1128/jvi.76.15.7430-7443.2002.
Hoffmann, Markus, Hannah Kleine-Weber, Simon Schroeder, Nadine Krüger, Tanja Herrler, Sandra Erichsen, Tobias S. Schiergens, et al. 2020. "SARS-CoV-2 Cell Entry Depends on ACE2 and TMPRSS2 and Is Blocked by a Clinically Proven Protease Inhibitor." Cell 181 (2): 271-280.e8. https://doi.org/10.1016/j.cell.2020.02.052.
Huang, I-Chueh, Berend Jan Bosch, Fang Li, Wenhui Li, Kyoung Hoa Lee, Sorina Ghiran, Natalya Vasilieva, et al. 2006. "SARS Coronavirus, but Not Human Coronavirus NL63, Utilizes Cathepsin L to Infect ACE2-Expressing Cells." The Journal of Biological Chemistry 281 (6): 3198-3203. https://doi.org/10.1074/jbc.M508381200.
Jané-Valbuena, Judit, Laura A Breun, Leslie A Schiff, and Max L Nibert. 2002. "Sites and Determinants of Early Cleavages in the Proteolytic Processing Pathway of Reovirus Surface Protein Sigma3." Journal of Virology 76 (10): 5184-97. https://doi.org/10.1128/jvi.76.10.5184-5197.2002.
Kawase, Miyuki, Kazuya Shirato, Shutoku Matsuyama, and Fumihiro Taguchi. 2009. "Protease-Mediated Entry via the Endosome of Human Coronavirus 229E." Journal of Virology 83 (2): 712-21. https://doi.org/10.1128/jvi.01933-08.
Licastro, Danilo, Sreejith Rajasekharan, Simeone Dal Monego, Ludovica Segat, Pierlanfranco D'Agaro, and Alessandro Marcello. 2020. "Isolation and Full-Length Genome Characterization of SARS-CoV-2 from COVID-19 Cases in Northern Italy." Edited by Rozanne M. Sandri-Goldin. Journal of Virology 94 (11). https://doi.org/10.1128/JVI.00543-20.
Milani M, Donalisio M, Milan Bonotto R, Schneider E, Arduino I, Boni F, Lembo D, Marcello A, Mastrangelo E. Combined in silico docking and in vitro antiviral testing for drug repurposing identified lurasidone and elbasvir as SARS-CoV-2 and CoV-OC43 inhibitors. BioRxiv 2020. doi: https://doi.org/10.1101/2020.11.12.379958.
Mirković, Bojana, Miha Renko, Samo Turk, Izidor Sosič, Zala Jevnikar, Nataša Obermajer, Dušan Turk, Stanislav Gobec, and Janko Kos. 2011. "Novel Mechanism of Cathepsin B Inhibition by Antibiotic Nitroxoline and Related Compounds." ChemMedChem 6 (8): 1351-56. https://doi.org/10.1002/cmdc.201100098.
Mitrović, Ana, Izidor Sosič, Špela Kos, Urša Lampreht Tratar, Barbara Breznik, Simona Kranjc, Bojana Mirković, et al. 2017. "Addition of 2-(Ethylamino)Acetonitrile Group to Nitroxoline Results in Significantly Improved Anti-Tumor Activityin Vitroandin Vivo." Oncotarget 8 (35): 59136-47. https://doi.org/10.18632/oncotarget.19296.
Nanbo, Asuka, Masaki Imai, Shinji Watanabe, Takeshi Noda, Kei Takahashi, Gabriele Neumann, Peter Halfmann, and Yoshihiro Kawaoka. 2010. "Ebolavirus Is Internalized into Host Cells via Macropinocytosis in a Viral Glycoprotein-Dependent Manner." Edited by Félix A. Rey. PLoS Pathogens 6 (9): e1001121. https://doi.org/10.1371/journal.ppat.1001121.
Pišlar, Anja, Ana Mitrovic, Jerica Sabotič, Urša Pečar Fonovic, Milica Perišic Nanut, Tanja Jakoš, Emanuela Senjor, and Janko Kos. 2020. "The Role of Cysteine Peptidases in Coronavirus Cell Entry and Replication: The Therapeutic Potential of Cathepsin Inhibitors." PLoS Pathogens 16 (11): 1-23. https://doi.org/10.1371/journal.ppat.1009013.
Rajasekharan, Sreejith, Rafaela Milan Bonotto, Yvette Kazungu, Lais Nascimento Alves, Monica Poggianella, Pamela Martinez Orellana, Natasa Skoko, Sulena Polez, and Alessandro Marcello. 2020. "Repurposing of Miglustat to Inhibit the Coronavirus Severe Acquired Respiratory Syndrome SARS-CoV-2." https://doi.org/10.1101/2020.05.18.101691.
Rawlings, Neil D., Alan J. Barrett, Paul D. Thomas, Xiaosong Huang, Alex Bateman, and Robert D. Finn. 2018. "The MEROPS Database of Proteolytic Enzymes, Their Substrates and Inhibitors in 2017 and a Comparison with Peptidases in the PANTHER Database." Nucleic Acids Research 46 (D1): D624-32. https://doi.org/10.1093/nar/gkx1134.
Regan, Andrew D, Renata Shraybman, Rebecca D Cohen, and Gary R Whittaker. 2008. "Differential Role for Low PH and Cathepsin-Mediated Cleavage of the Viral Spike Protein during Entry of Serotype II Feline Coronaviruses." Veterinary Microbiology 132 (3-4): 235-48. https://doi.org/10.1016/j.vetmic.2008.05.019.
Shirato, Kazuya, Miyuki Kawase, and Shutoku Matsuyama. 2013. "Middle East Respiratory Syndrome Coronavirus Infection Mediated by the Transmembrane Serine Protease TMPRSS2." Journal of Virology 87 (23): 12552-61. https://doi.org/10.1128/JVI.01890-13.
Siddhi et al. 2020. "Tackling SARS-CoV-2: Proposed targets and repurposed drugs." Future Medicinal Chemistry (12) 1579-1601.
Simmons, Graham, Dhaval N Gosalia, Andrew J Rennekamp, Jacqueline D Reeves, Scott L Diamond, and Paul Bates. 2005. "Inhibitors of Cathepsin L Prevent Severe Acute Respiratory Syndrome Coronavirus Entry." Proceedings of the National Academy of Sciences of the United States of America 102 (33): 11876-81. https://doi.org/10.1073/pnas.0505577102.
Simmons, Graham, Pawel Zmora, Stefanie Gierer, Adeline Heurich, and Stefan Pöhlmann. 2013. "Proteolytic Activation of the SARS-Coronavirus Spike Protein: Cutting Enzymes at the Cutting Edge of Antiviral Research." Antiviral Research 100 (3): 605-14. https://doi.org/10.1016/j.antiviral.2013.09.028.
Sosič, Izidor, Bojana Mirković, Katharina Arenz, Bogdan Štefane, Janko Kos, and Stanislav Gobec. 2013. "Development of New Cathepsin B Inhibitors: Combining Bioisosteric Replacements and Structure-Based Design to Explore the Structure-Activity Relationships of Nitroxoline Derivatives." Journal of Medicinal Chemistry 56 (2): 521-33. https://doi.org/10.1021/jm301544x.
Sosič, Izidor, Ana Mitrović, Hrvoje Ćurić, Damijan Knez, Helena Brodnik Žugelj, Bogdan Štefane, Janko Kos, and Stanislav Gobec. 2018. "Cathepsin B Inhibitors: Further Exploration of the Nitroxoline Core." Bioorganic & Medicinal Chemistry Letters 28 (7): 1239-47. https://doi.org/10.1016/j.bmcl.2018.02.042.
Turk, Boris, Dušan Turk, and Vito Turk. 2000. "Lysosomal Cysteine Proteases: More than Scavengers." Biochimica et Biophysica Acta - Protein Structure and Molecular Enzymology. https://doi.org/10.1016/S0167-4838(99)00263-0.
Voronin, V. G., I. D. Petrova, A. N. Leksin, and B. V. Shemeryankin. 1976. "Synthesis of 5-Nitro-8-Hydroxyquinoline." Pharmaceutical Chemistry Journal 10 (9): 1215-17. https://doi.org/10.1007/BF01156279.

## Claims

1. A compound having general formula (**I**), 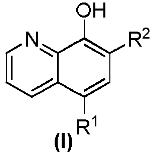 wherein:
R¹ is -NO₂;
R² is -H, 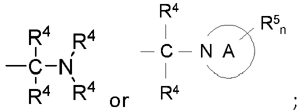
wherein A is a saturated or an unsaturated 3 to 8-membered heterocycle optionally comprising one or more further heteroatoms selected from N, O, S, and P, the heterocycle being optionally substituted with one or more substituents independently selected from R⁵, wherein n = 0 to 5;
R³ is -H, C₁₋₆ branched or linear alkyl optionally substituted with one or more substituents independently selected from R⁵, C₃-C₆ cycloalkyl optionally substituted with one or more substituents independently selected from R⁵;
R⁴ is -H; C₁₋₆ branched or linear alkyl optionally substituted with one or more substituents independently selected from R⁵ and optionally comprising one or more heteroatoms selected from N, S, O, P and optionally substituted with -N(R³)₂, C₃-C₆ cycloalkyl optionally comprising one or more heteroatoms selected from N, S, O, P and optionally substituted with -N(R³)₂, aryl optionally substituted with one or more substituents independently selected from R⁵, heteroaryl optionally substituted with one or more substituents independently selected from R⁵;
R⁵ is -H, halo, -NH₂, -OH, -CN, -NO₂, -COOH, -CONR⁴R⁴, -SO₂NR⁴R⁴, - SO₂R⁴, -CF₃, -NHSO₂R⁴, -NHCOR⁴, C₁₋₆ branched or linear alkyl, C₁₋₆ alkoxy, or -SR⁴;
wherein multiple occurrences of any of R³, R⁴ and R⁵ in the structure means that the respective residues are independently selected from the respective groups defined above;
or a pharmaceutically acceptable salt, hydrate or solvate thereof;
for use in the prevention and/or treatment of a disease caused by a virus of the coronavirus subfamily.

2. Compound for use according to claim 1, wherein R² is -H or

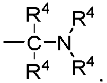

3. Compound for use according to claim 1 or 2, wherein R² is -H, morpholinomethyl, pyrrolidinomethyl, (4-methylpiperazin-1-yl)methyl, (4-hydroxypiperidin-1-yl)methyl, (4-methypiperidin-1-yl)methyl, (4-carboxypiperidin-1-yl)methyl, or (3-hydroxypiperidin-1-yl)methyl.

4. Compound for use according to any one of claims 1 to 3, wherein R² is -H.

5. Compound for use according to claim 1, wherein said compound is selected from the group consisting of 5-nitro-8-hydroxyquinoline, 7-(Morpholinomethyl)-5-nitro-8-hydroxyquinoline, 7-(Pyrrolidinomethyl)-5-nitro-8-hydroxyquinoline, 7-((4-Methylpiperazin-1-yl)methyl)-5-nitro-8-hydroxyquinoline, 7-((4-Hydroxypiperidin-1-yl)methyl)-5-nitro-8-hydroxyquinoline, 7-((4-Methypiperidin-1-yl)methyl)-5-nitro-8-hydroxyquinoline, 7-((4-Carboxypiperidin-1-yl)methyl)-5-nitro-8-hydroxyquinoline and 7-((3-Hydroxypiperidin-1-yl)methyl)-5-nitro-8-hydroxyquinoline.

6. Compound for use according to claim 1, wherein said compound is 5-nitro-8-hydroxyquinoline.

7. Compound for use according to any one of claims 1 to 6, wherein the disease is caused by a corona virus selected from severe acute respiratory syndrome related coronavirus, Middle East respiratory syndrome related coronavirus, HCoV-NL63, HCoV-HKU1, HCoV-229E and HCoV-OC43.

8. Compound for use according to any one of claims 1 to 7, wherein the disease is caused by SARS-CoV-2.

9. Compound for use according to any one of claims 1 to 8, wherein the disease is COVID-19.

10. Compound for use according to any one of claims 1 to 9, wherein the compound is for use in the prevention and/or treatment of said disease in a human.

11. Pharmaceutical composition, which comprises a compound having general formula (I) as defined in any one of claims 1 to 6 and a pharmaceutically acceptable carrier, excipient and/or diluent, for use in the prevention and/or treatment of a disease caused by a virus of the coronavirus subfamily.

12. Pharmaceutical composition for use according to claim 11, wherein the disease is caused by a corona virus selected from severe acute respiratory syndrome related coronavirus, Middle East respiratory syndrome related coronavirus, HCoV-NL63, HCoV-HKU1, HCoV-229E and HCoV-OC43.

13. Pharmaceutical composition for use according to any one of claims 11 to 12, wherein the disease is caused by SARS-CoV-2.

14. Pharmaceutical composition for use according to any one of claims 11 to 13, wherein the disease is COVID-19.

15. Pharmaceutical composition for use according to any one of claims 11 to 14, wherein the pharmaceutical composition is for use in the prevention and/or treatment of said disease in a human.

## Patentansprüche

1. Verbindung mit einer allgemeinen Formel (I), 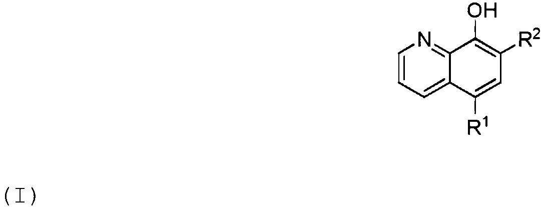 wobei:
R¹ -NO₂ ist; ;
R² -H, 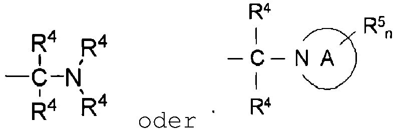 ist
wobei A ein gesättigter oder ungesättigter 3- bis 8-gliedriger Heterozyklus ist, der optional ein oder mehrere weitere Heteroatome umfasst, die aus N, O, S und P ausgewählt sind, wobei der Heterozyklus optional mit einem oder mehreren Substituenten substituiert ist, die unabhängig aus R⁵ ausgewählt sind, wobei n 0 bis 5 ist;
R³ -H, verzweigtes oder lineares C₁₋₆-Alkyl, das optional mit einem oder mehreren Substituenten substituiert ist, die unabhängig aus R⁵ ausgewählt sind, C₃-C₆-Cycloalkyl, das optional mit einem oder mehreren Substituenten substituiert ist, die unabhängig aus R⁵ ausgewählt sind, ist;
R⁴ -H; verzweigtes oder lineares C₁-₆-Alkyl, das optional mit einem oder mehreren Substituenten substituiert ist, die unabhängig aus R⁵ ausgewählt sind, und optional ein oder mehrere Heteroatome umfassend, ausgewählt aus N, S, O, P, und optional mit -N(R₃)₂ substituiert, C₃-C₆-Cycloalkyl, das optional ein oder mehrere Heteroatome umfasst, ausgewählt aus N, S, O, P, und optional mit -N(R₃)₂ substituiert ist, Aryl, das optional mit einem oder mehreren Substituenten substituiert ist, die unabhängig aus R⁵ ausgewählt sind, Heteroaryl, das optional mit einem oder mehreren Substituenten substituiert ist, die unabhängig aus R⁵ ausgewählt sind, ist;
R⁵ -H, Halogen, -NH₂, -OH, -CN, -NO₂, -COOH, -CONR⁴R⁴, -SO₂NR⁴R⁴, - SO₂R⁴, -CF₃, -NHSO₂R⁴, -NHCOR⁴, verzweigtes oder lineares C₁₋₆-Alkyl, C₁₋₆-Alkoxy oder -SR⁴ ist;
wobei mehrere Vorkommen von R³, R⁴ und R⁵ in der Struktur bedeuten, dass die jeweiligen Reste unabhängig aus den jeweils oben definierten Gruppen ausgewählt sind;
oder ein pharmazeutisch akzeptables Salz, Hydrat oder Solvat davon;
zur Verwendung bei der Vorbeugung und/oder Behandlung einer durch ein Virus der Coronavirus-Unterfamilie verursachten Krankheit.

2. Verbindung zur Verwendung nach Anspruch 1, wobei R² -H oder 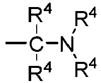 ist.

3. Verbindung zur Verwendung nach Anspruch 1 oder 2, wobei R² H, Morpholinomethyl, Pyrrolidinomethyl, (4-Methylpiperazin-1-yl)methyl, (4-Hydroxypiperidin-1-yl)methyl, (4-Methypiperidin-1-yl)methyl, (4-Carboxypiperidin-1-yl)methyl oder (3-Hydroxypiperidin-1-yl)methyl ist.

4. Verbindung zur Verwendung nach einem der Ansprüche 1 bis 3, wobei R² -H ist.

5. Verbindung zur Verwendung nach Anspruch 1, wobei die Verbindung aus der Gruppe ausgewählt ist, bestehend aus 5-Nitro-8-hydroxychinolin, 7-(Morpholinomethyl)-5-nitro-8-hydroxychinolin, 7-(Pyrrolidinomethyl)-5-nitro-8-hydroxychinolin, 7-((4-Methylpiperazin-1-yl)methyl)-5-nitro-8-hydroxychinolin, 7-((4-Hydroxypiperidin-1-yl)methyl)-5-nitro-8-hydroxychinolin, 7-((4-Methypiperidin-1-yl)methyl)-5-nitro-8-hydroxychinolin, 7-((4-Carboxypiperidin-1-yl)methyl)-5-nitro-8-hydroxychinolin und 7-((3-Hydroxypiperidin-1-yl)methyl)-5-nitro-8-hydroxychinolin.

6. Verbindung zur Verwendung nach Anspruch 1, wobei die Verbindung 5-Nitro-8-hydroxychinolin ist.

7. Verbindung zur Verwendung nach einem der Ansprüche 1 bis 6, wobei die Krankheit durch ein Coronavirus verursacht ist, ausgewählt aus mit schwerem akutem Atemwegssyndrom assoziiertem Coronavirus, mit schwerem Nahost-Atemwegssyndrom assoziiertem Coronavirus HCoV-NL63, HCoV-HKU1, HCoV-229E und HCoV-OC43.

8. Verbindung zur Verwendung nach einem der Ansprüche 1 bis 7, wobei die Krankheit durch SARS-CoV-2 verursacht ist.

9. Verbindung zur Verwendung nach einem der Ansprüche 1 bis 8, wobei die Krankheit COVID-19 ist.

10. Verbindung zur Verwendung nach einem der Ansprüche 1 bis 9, wobei die Verbindung zur Verwendung bei der Vorbeugung und/oder Behandlung der Krankheit bei einem Menschen bestimmt ist.

11. Pharmazeutische Zusammensetzung, die eine Verbindung mit der allgemeinen Formel (I) nach einem der Ansprüche 1 bis 6 und einen pharmazeutisch akzeptablen Träger, Hilfsstoff und/oder Verdünner umfasst, zur Verwendung bei der Vorbeugung und/oder Behandlung einer durch ein Virus der Coronavirus-Unterfamilie verursachten Krankheit.

12. Pharmazeutische Zusammensetzung zur Verwendung nach Anspruch 11, wobei die Krankheit durch ein Coronavirus verursacht ist, ausgewählt aus mit schwerem akutem Atemwegssyndrom assoziiertem Coronavirus, mit schwerem Nahost-Atemwegssyndrom assoziiertem Coronavirus HCoV-NL63, HCoV-HKU1, HCoV-229E und HCoV-OC43.

13. Pharmazeutische Zusammensetzung zur Verwendung nach einem der Ansprüche 11 bis 12, wobei die Krankheit durch SARS-CoV-2 verursacht ist.

14. Pharmazeutische Zusammensetzung zur Verwendung nach einem der Ansprüche 11 bis 13, wobei die Krankheit COVID-19 ist.

15. Pharmazeutische Zusammensetzung zur Verwendung nach einem der Ansprüche 11 bis 14, wobei die pharmazeutische Zusammensetzung zur Verwendung bei der Vorbeugung und/oder Behandlung der Krankheit bei einem Menschen bestimmt ist.

## Revendications

1. Composé répondant à la formule générale (I), 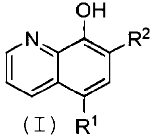 dans lequel :
R¹ est -NO₂ ;
R² est -H ; 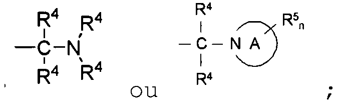
dans lequel A est un hétérocycle de 3 à 8 chaînons saturé ou insaturé comprenant éventuellement un ou plusieurs hétéroatomes supplémentaires choisis parmi N, O, S, et P, l'hétérocycle étant éventuellement substitué par un ou plusieurs substituants choisis indépendamment parmi R⁵, où n = 0 à 5 ;
R³ est -H, un alkyle linéaire ou ramifié en C₁₋₆ éventuellement substitué par un ou plusieurs substituants choisis indépendamment parmi R⁵, un cycloalkyle en C₃-C₆ éventuellement substitué par un ou plusieurs substituants choisis indépendamment parmi R⁵ ;
R⁴ est -H ; un alkyle linéaire ou ramifié en C₁₋₆ éventuellement substitué par un ou plusieurs substituants choisis indépendamment parmi R⁵ et comprenant éventuellement un ou plusieurs hétéroatomes choisis parmi N, S, O, P et éventuellement substitué par -N(R³)₂, un cycloalkyle en C₃-C₆ comprenant éventuellement un ou plusieurs hétéroatomes choisis parmi N, S, O, P et éventuellement substitué par -N(R³)₂, un aryle éventuellement substitué par un ou plusieurs substituants choisis indépendamment parmi R⁵, un hétéroaryle éventuellement substitué par un ou plusieurs substituants choisis indépendamment parmi R⁵ ;
R⁵ est -H, halo, -NH₂, -OH, -CN, -NO₂, -COOH, -CONR⁴R⁴, -SO₂NR⁴R⁴, -SO₂R⁴, -CF₃, -NHSO₂R⁴, -NHCOR⁴, un alkyle linéaire ou ramifié en C₁₋₆, un alcoxy en C₁₋₆, ou -SR⁴ ;
dans lequel de multiples occurrences de l'un quelconque de R³, R⁴ et R⁵ dans la structure signifient que les résidus respectifs sont choisis indépendamment parmi les groupes respectifs définis ci-dessus ;
ou un sel, hydrate ou solvate pharmaceutiquement acceptable de celui-ci ;
pour une utilisation dans la prévention et/ou le traitement d'une maladie causée par un virus de la sous-famille des coronavirus.

2. Composé pour une utilisation selon la revendication 1, dans lequel R² est -H ou

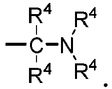

3. Composé pour une utilisation selon la revendication 1 ou 2, dans lequel R² est H, morpholinométhyle, pyrrolidinométhyle, (4-méthylpipérazin-1-yl)méthyle, (4-hydroxypipéridin-1-yl)méthyle, (4-méthylpipéridin-1-yl)méthyle, (4-carboxypipéridin-1-yl)méthyle, ou (3-hydroxypipéridin-1-yl)méthyle.

4. Composé pour une utilisation selon l'une quelconque des revendications 1 à 3, dans lequel R² est -H.

5. Composé pour une utilisation selon la revendication 1, dans lequel ledit composé est choisi dans le groupe constitué par la 5-nitro-8-hydroxyquinoléine, la 7-(morpholinométhyl)-5-nitro-8-hydroxyquinoléine, la 7-(pyrrolidinométhyl)-5-nitro-8-hydroxyquinoléine, la 7-((4-méthylpipérazin-1-yl)méthyl)-5-nitro-8-hydroxyquinoléine, la 7-((4-hydroxypipéridin-1-yl)méthyl)-5-nitro-8-hydroxyquinoléine, la 7-((4-méthylpipéridin-1-yl)méthyl)-5-nitro-8-hydroxyquinoléine, la 7-((4-carboxypipéridin-1-yl)méthyl)-5-nitro-8-hydroxyquinoléine et la 7-((3-hydroxypipéridin-1-yl)méthyl)-5-nitro-8-hydroxyquinoléine.

6. Composé pour une utilisation selon la revendication 1, dans lequel ledit composé est la 5-nitro-8-hydroxyquinoléine.

7. Composé pour une utilisation selon l'une quelconque des revendications 1 à 6, dans lequel la maladie est causée par un coronavirus choisi parmi le coronavirus du syndrome respiratoire aigu sévère, le coronavirus du syndrome respiratoire du Moyen-Orient, HCoV-NL63, HCoV-HKU1, HCoV-229E et HCoV-OC43.

8. Composé pour une utilisation selon l'une quelconque des revendications 1 à 7, dans lequel la maladie est causée par le SARS-CoV-2.

9. Composé pour une utilisation selon l'une quelconque des revendications 1 à 8, dans lequel la maladie est la COVID-19.

10. Composé pour une utilisation selon l'une quelconque des revendications 1 à 9, dans lequel le composé est destiné à une utilisation dans la prévention et/ou le traitement de ladite maladie chez un humain.

11. Composition pharmaceutique, qui comprend un composé répondant à la formule générale (I) tel que défini dans l'une quelconque des revendications 1 à 6 et un support, excipient et/ou diluant pharmaceutiquement acceptable, pour une utilisation dans la prévention et/ou le traitement d'une maladie causée par un virus de la sous-famille des coronavirus.

12. Composition pharmaceutique pour une utilisation selon la revendication 11, dans laquelle la maladie est causée par un coronavirus choisi parmi le coronavirus du syndrome respiratoire aigu sévère, le coronavirus du syndrome respiratoire du Moyen-Orient, HCoV-NL63, HCoV-HKU1, HCoV-229E et HCoV-OC43.

13. Composition pharmaceutique pour une utilisation selon l'une quelconque des revendications 11 à 12, dans laquelle la maladie est causée par le SARS-CoV-2.

14. Composition pharmaceutique pour une utilisation selon l'une quelconque des revendications 11 à 13, dans laquelle la maladie est la COVID-19.

15. Composition pharmaceutique pour une utilisation selon l'une quelconque des revendications 11 à 14, dans laquelle la composition pharmaceutique est destinée à une utilisation dans la prévention et/ou le traitement de ladite maladie chez un humain.
